# EUROPÄISCHE PATENTSCHRIFT

(11) **EP 1 584 347 B1**
(45) Veröffentlichungstag und Bekanntmachung des Hinweises auf die Patenterteilung: **14.06.2006**
(21) Anmeldenummer: 04007751.3
(22) Anmeldetag: 31.03.2004
(51) Int. Cl.: A61M 39/10, F16L 37/00, F16L 37/47

(54) **Clip**
Clip
Pince

(43) Veröffentlichungstag der Anmeldung: 12.10.2005
(73) Patentinhaber: Codan Holding GmbH, 23738 Lensahn (DE)
(72) Erfinder: Hoogers, Cornelius F., 7271 W.E.Borculo (NL)
(74) Vertreter: Fleck, Thomas

(56) Entgegenhaltungen:
- EP-A- 1 277 490
- DE-U- 9 417 075
- GB-A- 2 334 315
- US-A- 6 096 024

## Beschreibung

Die Erfindung betrifft einen Clip, Klammer oder Klemme zum Einsatz im medizinischen Bereich. Derartige Clips gibt es in den unterschiedlichsten Ausführungsformen, beispielsweise wird verwiesen auf die DE-U 94 17 075, GB-A-2 334 315 und US-A-6,096,024.

Die erstgenannte Druckschrift beschreibt einen Clip, der auf einen Drehverschlussstopfen einer Einweg-Braunüle zur Fehlbediensicherung aufgesetzt wird.

Die GB-A-2 334 315 zeigt eine Kupplung zum lösbaren Verbinden eines Urinentsorgungsschlauches mit einem Urinsammelbeutel.

Die US-A-6,096,024 beschreibt einen Stumpfnadelverbinder zum Überführen von med. Flüssigkeiten über kompakte Einspritzöffnungen in Leitungen während intravenöser Therapieanwendungen. Der Verbinder weist eine Einschnappklinke auf, die schwenkbar mit einer Schutzabdeckung verbunden ist.

Die Erfindung befasst sich jedoch insbesondere mit einem Clip als Verbindungs- oder Befestigungselement zwischen einem einen Einstechdorn aufweisenden Medikamentenmisch- und Verabreichungsset und einem Infusionsbeutel mit Anschlussstutzen.

Ein derartiges Medikamentenmisch- und Verabreichungsset wurde von der vorliegenden Anmelderin unter der europäischen Patentanmeldung 01 117 239.2 (EP-A-1 277 490) angemeldet und wird für die Zubereitung und Verabreichung von gefährlichen Medikamenten wie Zytostatika und Antibiotika verwendet. Der Einstechdorn ist von den Abmessungen her standardisiert, während es für die Anschlussstutzen der Infusionsbeutel keine standardisierten Designvorgaben gibt. Eine Vielzahl der Infusionsbeutel wird aus flexiblen, PVC-haltigen Materialien hergestellt. Die Verbindung des Anschlussstutzens mit dem Einstechdorn wird als zufrieden stellend angesehen.

Anders verhält es sich hingegen, wenn die Infusionsbeutel mit ihrem Anschlussstutzen aus PVC-freien Materialien bestehen, die aus sehr harten, gering flexiblen Werkstoffen hergestellt werden. Hier ist die Verbindung zwischen Einstechdorn und Anschlussstutzen teilweise unzureichend, da sich der Einstechdorn ohne großen Kraftaufwand aus dem Infusionsbeutel ziehen lässt bzw. sich von ihm löst, was auf dem Transport der vorbereiteten Medikamenteneinheit zum Applikationsort oder auch bei der Verabreichung des Medikamentes selbst geschehen kann. Eine Leckage mit den gefährlichen Medikamenten stellt eine erhebliche Gefährdung für den Patienten und den Anwender dar.

Ein weiteres Problem ist dem Einstechdorn des Medikamentenmisch- und Verabreichungssets eigen, an dem sich ein Zugangsport befindet, über den das Medikament in den Infusionsbeutel appliziert wird. Nach der Zubereitung wird dieser Zugangsport mit einem Verschlussstopfen wieder verschlossen. Es besteht aber weiterhin die Möglichkeit, dass an diesem Zugangsport später durch nicht zuständiges oder sachunkundiges Personal manipuliert werden kann. Aufgrund dessen besteht Bedarf an einer sicheren Verbindung der genannten Medikamenteneinrichtungen, die nicht nur Leckagen, sondern auch unsachgemäße Manipulationen am Zugangsport gleicherma-ßen verhindert.

Eine solche Verbindung, die sicher und permanent installiert wird, liefert der erfindungsgemäße Clip gemäß Anspruch 1, der nach Zubereitung des Medikamentes zwischen dem Infusionsbeutel mit Anschlussstutzen und dem Medikamentenmisch- und Verabreichungsset fixiert und arretiert wird.

Durch den erfindungsgemäßen Aufbau des Clips mit einem haubenförmigen Abdeckteil über dem Zugangsport oder der Zuspritzöffnung wird sichergestellt, dass letzterer permanent verschlossen bleibt. Nach der Montage des erfindungsgemäßen Clips ist die Verbindung zwischen dem Infusionsbeutel einerseits und dem Medikamentenmisch- und Verabreichungsset andererseits durch diese Arretierung gesichert und der Zugangsport gleichermaßen geschützt. Es dürfte einleuchten, dass die Verbindung nicht wieder rückgängig gemacht werden kann.

Weitere Vorteile und Merkmale gehen aus den Unteransprüchen hervor, die auch gemeinsam mit dem Hauptanspruch von erfinderischer Bedeutung sein können. Im folgenden wird zum besseren Verständnis der Erfindung ein bevorzugtes Ausführungsbeispiel beschrieben, auf das die Erfindung jedoch nicht beschränkt ist.

Es zeigt:
- Fig. 1: eine schematische Querschnittsgesamtansicht des erfindungsgemäßen Clips im Gebrauchszustand, arretiert am Einstechdorn eines Medikamentenmisch- und Verabreichungssets und dem Anschlussstutzen eines angerissen dargestellten Infusionsbeutels;
- Fig. 2: Seitenansicht der in Fig. 1 gezeigten Elemente;
- Fig. 3 und 4: vergrößerte schematische Querschnittsansichten des erfindungsgemäßen Clips beim Einsetzen bzw. festlegen desselben;
- Fig. 5 und 6: schematische Ansichten des in Figuren 3 und 4 gezeigten erfindungsgemäßen Clips von oben; und
- Fig. 7: eine schematische Seitenansicht des in den Figuren 4 und 6 gezeigten arretierten erfindungsgemäßen Clips.

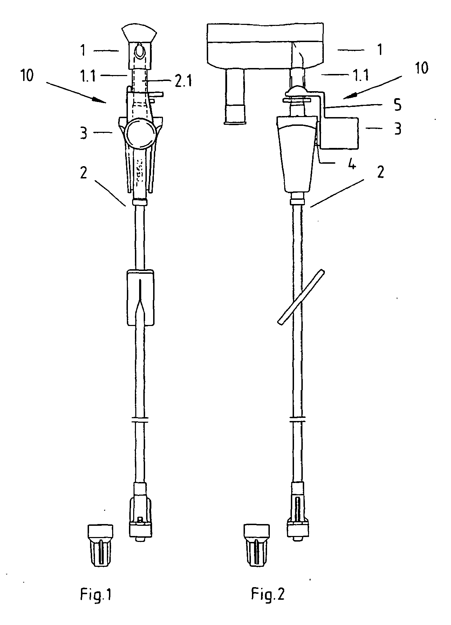

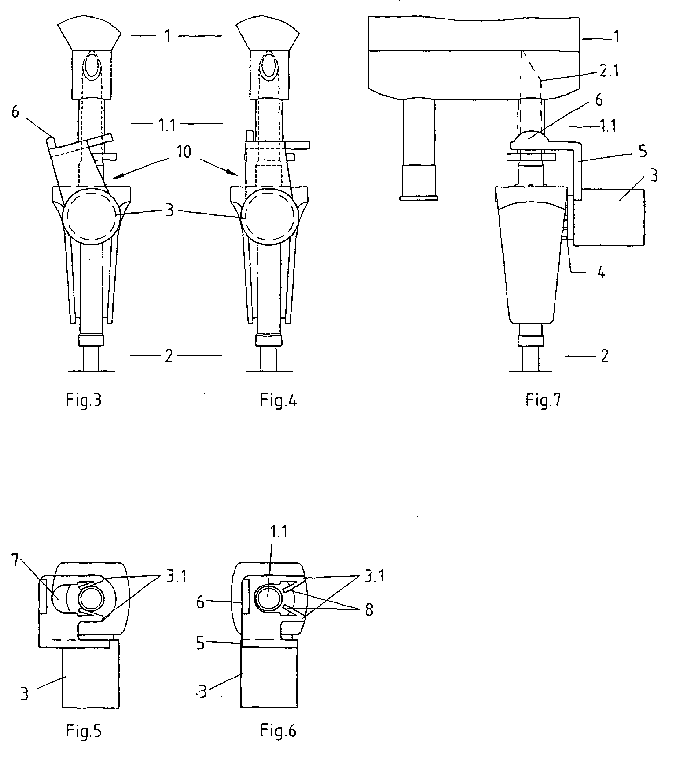

In den Figuren 1 und 2 ist der erfindungsgemäße Clip allgemein mit 10 bezeichnet. Er ist in seiner festgelegten Form gezeigt, wie er einen Einstechdorn 2.1 eines Medikamentenmisch- und Verabreichungssets 2 mit einem Anschlussstutzen 1.1 eines Infusionsbeutels 1 permanent verbindet. Der Infusionsbeutel 1 ist nur im Anschnitt gezeichnet. Vom erfindungsgemäßen Aufbau her weist der erfindungsgemäße Clip 10 drei Konstruktionsmerkmale auf, nämlich ein haubenförmiges Abdeckteil 3 für einen Zugangsport 4, der in diesen beiden Figuren abgedeckt und somit nicht sichtbar ist. An seinem oberen Ende weist das U- und haubenförmige Abdeckteil 3 ein L-förmiges laschenartiges Verbindungsstück 5 auf, das ein Befestigungsteil 6 an seinem anderen Ende zum Festlegen am Anschlussstutzen 1.1 aufweist. Einerseits ist hierdurch eine permanente sichere Verbindung geschaffen, die ein zufälliges oder unbedachtes Lösen der genannten Elemente voneinander verhindert. Andererseits werden gleichzeitig Manipulationen am Zugangsport oder der Zuspritzöffnung 4 (siehe Fig. 7) verhindert.

In den übrigen Figuren werden dieselben Teile mit denselben Bezugszeichen bezeichnet, jedoch andere Ansichten, auch in vergrößerter Form gezeigt.

Insbesondere ist der Fig. 3 zu entnehmen, wie der erfindungsgemäße Clip zunächst mit seinem haubenförmigen Abdeckteil auf dem mit einem Stopfen verschlossenen Zugangsport 4 aufgesetzt wird, wobei das Befestigungsteil 6 mit dem vorderen Teil seines U-förmigen Kanals 7 den senkrecht dazu verlaufenden Anschlussstutzen 1.1 aufnimmt und dann nach rechts verschwenkt wird, wie es in Fig. 4 gezeigt ist, wo der Anschlussstutzen 1.1 am hinteren Ende des U-förmigen Kanals 7 an der Wandung anliegt und so seinen endgültigen Arretierungszustand erreicht.

Die gleiche Montage des Clips ist aus der Sicht von oben in den Figuren 5 und 6 gezeigt, wo der Anschlussstutzen 1.1 zunächst mit den zurück verlaufenden federnden Stegen 8 zur Anlage gelangt (Fig. 5) und dann bei der weiteren Bewegung des erfindungsgemäßen Clips 10 nach rechts in seine endgültige hintere Position gelangt, aus der er nicht mehr herausgezogen werden kann, da dann die Stege 8 den Weg versperren.

In Fig. 7 ist dann der erfindungsgemäße Clip noch einmal in der seitlichen Ansicht gezeigt, wobei wiederum der Zugangsport 4 durch das haubenförmige Abdeckteil 3 verschlossen ist und das am Ende des L-förmigen Verbindungsstücks 5 liegende Befestigungsteil 6 den Anschlussstutzen 1.1 des Infusionsbeutels 1 formschlüssig umgibt und arretiert, so dass sämtliche Elemente permanent miteinander verbunden sind. In Fig. 7 und auch den Figuren 3 und 4 ist der Einstechdorn 2.1 in gestrichelter Form dargestellt, wie er durch den Anschlussstutzen 1.1 hindurch verläuft und mit seiner Spitze im Infusionsbeutel 1 endet.

## Patentansprüche

1. Clip (10) dazu angepasst, einen Einstechdorn (2.1) eines Medikamentenmisch- und Verabreichungssets (2) mit einem Anschlussstutzen (1.1) eines Infusionsbeutels (1) permanent zu verbinden, umfassend ein haubenförmiges Abdeckteil (3), welches zum Aufsetzen auf einen Zugangsport (4) des Medikamentenmisch- und Verabreichungssets (2) angepasst ist und ein L-förmiges Verbindungsstück (5), das an seinem Ende ein Befestigungsteil (6) zum Festlegen am Anschlussstutzen (1.1) aufweist.

2. Clip (10) nach Anspruch 1, **dadurch gekennzeichnet, dass** das Befestigungsteil (6) einen U-förmigen Kanal (7) mit gegenüberliegenden Schenkeln (3.1) zur passgerechten Aufnahme des senkrecht dazu verlaufenden Anschlussstutzens (1.1) besitzt, wobei an den Schenkelenden jeweils ein nach innen zurück verlaufender, federnder Steg (8) angeordnet ist.

3. Clip (10) nach Anspruch 1 oder 2, **dadurch gekennzeichnet, dass** die eingesetzten Infusionsbeutel (1) aus PVC-freien Materialien bestehen.

4. Clip (10) nach Anspruch 1 bis 3, **dadurch gekennzeichnet, dass** er aus Polypropylen-Kunststoff besteht.

## Claims

1. Clip (10) designed for permanently linking a perforating mandrel (2.1) of a medicament mixing and administration set (2) with a spigot (1.1) of an infusion bag (1), comprising a hood-shaped covering part (3) for placing on an access port (4) of the medicament mixing and administration set (2) and a L-shaped connecting piece (5) provided at its end with a fastening part (6) for fixing to the spigot (1.1).

2. Clip (10) according to claim 1, **characterized in that** the fastening part (6) has a U-shaped duct (7) with facing legs (3.1) for the precise fitting reception of the spigot (1.1) at right angles thereto, an inwardly back-passing, resilient web (8) being located on each of the leg ends.

3. Clip (10) according to claim 1 or 2, **characterized in that** the infusion bags (1) used are of PVC-free materials.

4. Clip (10) according to claim 1 to 3, **characterized in that** it is made from polypropylene plastic.

## Revendications

1. Pince (10) adaptée pour relier de manière permanente un mandrin de perçage (2.1) d'un kit de mélange et d'administration de médicaments (2) à une tubulure de raccordement (1.1) d'une poche de perfusion (1), comprenant un élément de couverture en forme de capot (3) qui est adapté pour être posé sur un port d'accès (4) du kit de mélange et d'administration de médicaments (2) et un élément de liaison en forme de L (5) qui comporte à son extrémité une partie de fixation (6) à la tubulure de raccordement (1.1).

2. Pince (10) selon la revendication 1, **caractérisée en ce que** la partie de fixation (6) possède un canal en forme de U (7) avec des branches (3.1) qui se font face pour loger de manière ajustée la tubulure de raccordement (1.1) perpendiculaire à ces dernières, une bride à ressort (8) repliée vers l'intérieur étant disposée aux extrémités des branches.

3. Pince (10) selon la revendication 1 ou 2, **caractérisée en ce que** les poches de perfusion utilisées (1) sont en matériaux exempts de PVC.

4. Pince (10) selon les revendications 1 à 3, **caractérisée en ce qu'**elle est en matière plastique en polypropylène.
